Europäisches Patentamt

European Patent Office

Office européen des brevets

(19)

(11) Veröffentlichungsnummer: **0 116 325**
**B1**

# (12) EUROPÄISCHE PATENTSCHRIFT

(45) Veröffentlichungstag der Patentschrift:
**10.12.86**

(21) Anmeldenummer: **84100863.4**

(22) Anmeldetag: **27.01.84**

(51) Int. Cl.⁴: **A 61 M 5/14,** F 16 K 7/06

(54) Dosiervorrichtung für Flüssigkeiten für Infusions- oder Transfusionsgeräte.

(30) Priorität: **11.02.83 DE 3304831**

(43) Veröffentlichungstag der Anmeldung:
**22.08.84 Patentblatt 84/34**

(45) Bekanntmachung des Hinweises auf die Patenterteilung:
**10.12.86 Patentblatt 86/50**

(84) Benannte Vertragsstaaten:
**AT BE CH DE FR GB IT LI LU NL SE**

(56) Entgegenhaltungen:
**DE - A - 2 340 611**
**FR - A - 1 306 090**
**FR - A - 2 448 098**
**US - A - 3 497 175**

(73) Patentinhaber: **Matthias Faensen**
**Kleinmetallwaren-Fabrikation, Hauptstrasse 26,**
**D-5190 Stolberg-Gressenich (DE)**
Patentinhaber: **Iphas Pharma-Verpackung,**
**Römerstrasse 26, D-5190 Stolberg-Gressenich (DE)**

(72) Erfinder: **Mommer, Heinrich, Schevenhütter Strasse 27,**
**D-5190 Stolberg/Gressenich (DE)**

(74) Vertreter: **Selting, Günther, Dipl.-Ing. et al,**
**Deichmannhaus am Hauptbahnhof, D-5000 Köln 1 (DE)**

## Beschreibung

Die Erfindung betrifft eine Dosiervorrichtung für Flüssigkeiten für Infusions- oder Transfusionsgeräte mit einer durch ein Gehäuse hindurchgehenden flexiblen Flüssigkeitsleitung, die im Innern des Gehäuses ein Schlauchstück aus hochelastischem elastomerem Material enthält, und mit einem am Gehäuse abgestützten Drückelement zum Zusammendrücken des Schlauchstückes.

Bei der Durchführung von Infusionen oder Transfusionen wird üblicherweise ein die Infusions- oder Transfusionsflüssigkeit enthaltender Behälter mit nach unten weisender Öffnung und angeschlossener Flüssigkeitsleitung so hoch aufgehängt, dass der Flüssigkeitsdruck in der Leitung höher ist als der Venendruck des Patienten. Die Flüssigkeit wird zunächst einem Zwischenbehälter in der Flüssigkeitsleitung zugeführt und gelangt von dort zum Patienten. Aufgrund der unterschiedlichen Randbedingungen, z.B. des Venendruckes des Patienten, der Viskosität der Flüssigkeit, und der unterschiedlichen Füllhöhe des Flüssigkeitsbehälters sowie aufgrund therapeutisch notwendiger bestimmter Dosierungen der Flüssigkeit, ist es erforderlich, die Durchflussmenge der Flüssigkeit exakt zu regeln.

Bei einer bekannten Dosiervorrichtung für Flüssigkeiten bei intravenösen Infusionen (DE-A-2 340 611) weist eine Flüssigkeitsleitung einen Leitungsabschnitt aus elastischem Material auf, der mit einem an einem Gehäuse abgestützten Drückelement zusammengedrückt werden kann. Dabei ist das elastische Schlauchelement durch eine Öffnung des Gehäuses geführt, die grösser als der Querschnitt des Schlauchstückes ist. Die innere Öffnung des Schlauchstückes ist nicht kreisförmigen Querschnitts, sondern weist mehrere radial gleichmässig verteilte und axial verlaufende V-förmige Rillen auf. Ein derartiges Schlauchstück kann folglich selbst im restlos zusammengedrückten Zustand den Durchfluss nicht vollständig unterbinden. Ferner ist eine Verschiebung innerhalb des Gehäuses des Drückelementes möglich, da das Schlauchstück nicht am Gehäuse arretiert werden kann.

Bekannt sind Dosiervorrichtungen der eingangs genannten Art mit einer durch ein Gehäuse hindurchgehenden Flüssigkeitsleitung und einem Klemmelement, das aus einer in dem Gehäuse geführten und auf den Schlauchquerschnitt drückenden Rolle besteht (DE-A-2 637 495). Die Dosiermenge, die üblicherweise als die Anzahl der in den Zwischenbehälter pro Zeiteinheit fallenden Flüssigkeitstropfen ermittelt wird, lässt sich mit derartigen Vorrichtungen nicht über einen längeren Zeitraum konstant halten, da sich die üblicherweise verwendeten PVC-Schläuche infolge ihrer Neigung zum Kriechen (Kaltfliessvermögen) verformen können. Insbesondere treten Probleme auf, wenn die Dosiermenge wieder erhöht werden soll und die Flüssigkeitsleitung zuvor längere Zeit zusammengedrückt war, da das zähelastische und nicht hysteresefreie Relaxationsverhalten der Kunststoffleitung eine schnelle Rückkehr in den ursprünglichen Leitungsquerschnitt verhindert. Daher kann bei derartigen Dosiervorrichtungen oft selbst bei ständiger Regulierung eine konstante Dosierung nicht erreicht werden.

Bekannt ist ferner eine Dosiervorrichtung mit einem Gehäuse und einem in dem Gehäuse vorgesehenen im wesentlichen zylindrischen Ventilraum, zwei von dem Gehäuse bzw. dem Ventilraum radial nach entgegengesetzten Richtungen abstehenden, jeweils einen Kanal aufweisenden Anschlussstutzen, und einem mit einem Handgriff drehbaren Küken, das in dem Ventilraum abdichtend eingesetzt ist. In der Mantelfläche des Kükens ist ein Durchlass vorgesehen, der in Abhängigkeit von der Drehstellung des Kükens unterschiedlich grosse Durchflussquerschnitte zwischen den Anschlussstutzen freigibt (DE-A-2 735 955). Bei derartigen Dosiervorrichtungen muss ein hoher konstruktiver Aufwand betrieben werden, um eine sichere Abdichtung der Dosiervorrichtung gegen Lufteintritt zu gewährleisten.

Der Erfindung liegt die Aufgabe zugrunde, eine Dosiervorrichtung der eingangs genannten Art zu schaffen, die eine exakte, zeitkonstante und reproduzierbar einstellbare Dosierung von Flüssigkeiten auch bei einem Schlauchstück mit kreisförmigem Innenquerschnitt sowie ein Zuspritzen von Injektionsflüssigkeiten erlaubt.

Zur Lösung dieser Aufgabe ist erfindungsgemäss vorgesehen, dass das Schlauchstück auf einem Teil seiner Länge eine Verdickung aufweist, dass das Gehäuse an der Verdickung des Schlauchstückes gegen Längsverschiebung gesichert ist und dass das Drückelement ausserhalb des Bereiches der Verdickung an dem Schlauchstück angreift.

Die Flüssigkeitsleitung kann in der bisherigen Weise aus geeignetem Kunststoff hergestellt sein. Sie enthält an einer geeigneten Stelle ein elastomeres Schlauchstück, das von einem Gehäuse umgeben ist und das gemeinsam mit diesem Gehäuse, einem Drückelement und einer Stellvorrichtung die Dosiervorrichtung bildet. Das Schlauchstück weist eine hohe Elastizität und Rückstellkraft auf, so dass die Querschnittsfläche des Schlauchstückes immer bestrebt ist, ihren entspannten Ursprungszustand zu erreichen, soweit es das Regelelement der Dosiervorrichtung, hier ein Drückelement, zulässt. Eine Änderung der Position des Drückelementes bewirkt eine sofortige Änderung des Durchflussquerschnittes des Schlauchstückes, auch wenn das Drückelement zurückgestellt wurde. Auf diese Weise wird eine sehr hohe Dosiergenauigkeit erreicht, die eine weitgehend zeitkonstante Tropfgeschwindigkeit der Flüssigkeit erlaubt, und die reproduzierbar eingestellt werden kann. Die erfindungsgemässe Dosiervorrichtung garantiert — bei intravenösen Infusions- und Transfusionsgeräten ein wesentlicher Sicherheitsaspekt — in hohem Masse eine Dichtigkeit gegen Lufteintritt, weil die durchflussregelnde Vorrichtung, hier das Schlauchstück, aus einem Stück besteht. Die Dosiervorrichtung lässt sich kostengünstig herstellen. Das Gehäuse wird in einstückiger Fertigung als Spritzgussteil produziert. Das Gehäuse und die Stellvorrichtung sowie das Drückelement brauchen nicht sterilisiert zu werden, weil nur das Schlauchstück mit der Infusions- oder Transfusionsflüssigkeit in Berührung kommt, wodurch die Kosten der Sterilisation der Dosiervorrichtung und die Gefahren einer mangelhaften Sterilisation vermieden werden.

Aufgrund der Form des Schlauchstückes ist die Position des Gehäuses exakt fixiert. Ein wesentlicher Vorteil ist, dass das Schlauchstück als Zuspritzteil bekannt und handelsüblich ist und daher keine speziellen Teile notwendig sind. In der erfindungsgemässen Dosiervorrichtung kann das Schlauchstück ebenfalls zum Zuspritzen zusätzlicher Medikamente oder Flüssigkeiten mit einer Spritze verwendet werden. Dadurch, dass das Schlauchstück aus einem hochelastischen elastomeren Material besteht, ist die Dichtigkeit der Dosiervorrichtung auch nach dem Einstechen und Herausziehen der Nadelkanüle gewährleistet. Die Verdickung des Schlauchstückes ermöglicht einen grösseren Spielraum im Hohlraum des Schlauchstückes hinsichtlich der Einstichtiefe der Nadelkanüle und erhöht damit die Sicherheit der Vorrichtung.

Das Schlauchstück ist folglich von doppeltem Nutzen, indem es einerseits die Möglichkeit bietet, Medikamente oder Flüssigkeiten zuzuspritzen und indem es andererseits in Verbindung mit dem Drückelement die Durchflussmenge der Infusions- bzw. Transfusionsflüssigkeit dosiert. Für diese Aufgaben werden sonst zwei Schlauchabschnitte benötigt.

Ein bevorzugtes Ausführungsbeispiel der Erfindung ist dadurch gekennzeichnet, dass das Drückelement quer zur Längsrichtung des Schlauchstückes geführt ist und dass die gegen das Schlauchstück drückende Vorderseite des Drückelementes zur Bewegungsrichtung des Drückelementes schräggestellt ist. Eine derartige asymmetrische Verformung des Schlauchstückes hat den Vorteil, das Schlauchstück sukzessive von einer Seite her zu klemmen, wodurch verhindert wird, dass das Schlauchstück in einer anderen Querschnittsform, z.B. in Form einer Acht, trotz der Elastizität seine Verformung beibehält oder sprunghaft seine Querschnittsform ändert. Dies gewährleistet eine besonders exakte, reproduzierbar einstellbare Dosierung.

Eine vorteilhafte Weiterbildung der Erfindung ist dadurch gekennzeichnet, dass das Gehäuse einen zum Schlauchstück führenden Einstichkanal für eine Nadelkanüle aufweist. Auf diese Weise wird die Nadelkanüle einer bestimmten Stelle des Schlauchstückes zugeführt.

Eine bevorzugte Ausführungsform der Erfindung ist dadurch gekennzeichnet, dass der Einstichkanal unter einem spitzen Winkel zur Längsachse des Schlauchstückes verläuft. Dies hat den Vorteil, dass aufgrund der Führung der Nadelkanüle der günstigste Einstichwinkel eingehalten wird. Dadurch wird die Sicherheit beim Zuspritzen erhöht, da ein maximaler freier Weg für die Nadelspitze im Schlauchstück erzielt wird. Dies ist wichtig, wenn in Notfällen besonders schnell zugespritzt werden muss, da die Nadelspitze leichter und sicherer im Schlauchquerschnitt positioniert und nicht durch ein zu tiefes Einstechen auch noch die weitere Schlauchwand perforiert werden kann, wodurch die Zuspritzflüssigkeit nicht in den Blutkreislauf gelangen würde.

Bei einer vorteilhaften Weiterbildung der Erfindung ist vorgesehen, dass das Drückelement über eine manuell verstellbare selbsthemmende Stellvorrichtung bewegbar ist. Die Stellvorrichtung kann bevorzugt aus einer im Gehäuse gelagerten Gewindespindel bestehen. Mit einer Gewindespindel ist je nach Gewindesteigung eine besonders genaue und reproduzierbare Dosierung möglich.

Die Stellvorrichtung kann auch ein abnehmbares Griffteil haben. Dies erhöht wesentlich die Sicherheit gegen unerlaubtes Verstellen der Dosiervorrichtung, da sie bei abgenommenem Griffteil nicht verstellt werden kann.

Weitere vorteilhafte Ausgestaltungen der Erfindung sind den Ansprüchen 4, 8 und 9 zu entnehmen.

Im folgenden werden unter Bezugnahme auf die Zeichnungen Ausführungsbeispiele der Erfindung näher erläutert.

Es zeigen:

Fig. 1 einen Längsschnitt durch eine Dosiervorrichtung, mit einer Gewindespindel als Stellvorrichtung,

Fig. 2 eine Draufsicht auf diese Dosiervorrichtung,

Fig. 3 einen Schnitt entlang der Linie III-III in Fig. 1,

Fig. 4 eine perspektivische Explosionsdarstellung der Dosiervorrichtung gemäss Fig. 1 mit auseinandergeklappten Gehäusehälften,

Fig. 5 einen Längsschnitt durch eine Dosiervorrichtung mit einem Exzenter als Stellvorrichtung,

Fig. 6 eine Draufsicht der Dosiervorrichtung gemäss Fig. 5,

Fig. 7 einen Schnitt entlang der Linie VII-VII in Fig. 5,

Fig. 8 eine perspektivische Explosionsdarstellung der Dosiervorrichtung gemäss Fig. 5 mit auseinandergeklappten Gehäusehälften und

Fig. 9 eine perspektivische Darstellung einer Dosiervorrichtung mit einem Hebel als Stellvorrichtung mit auseinandergeklappten Gehäusehälften.

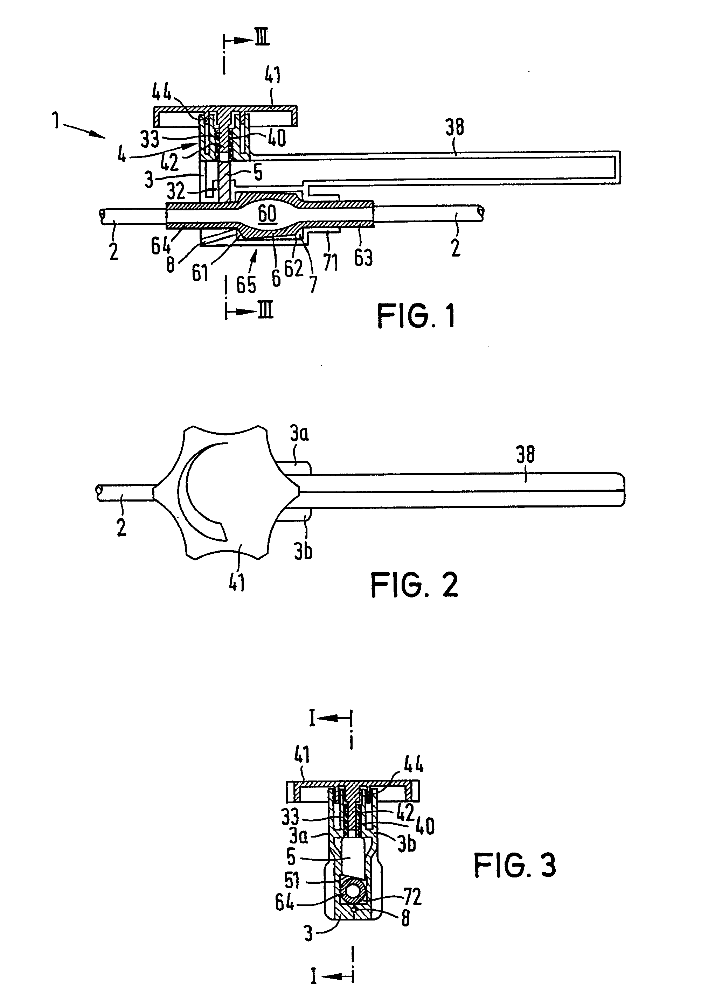

Die in den Figuren 1 bis 9 gezeigten Ausführungsbeispiele einer Dosiervorrichtung 1 sind in einer Infusions- oder Transfusionseinrichtung angeordnet. Die Dosiervorrichtung 1 in der Flüssigkeitsleitung 2 befindet sich folglich zwischen einem Infusionsbehälter und einem Patienten. Sie weist ein Gehäuse 3 zur Aufnahme einer Stellvorrichtung 4, eines Drückelementes 5 und eines in die Flüssigkeitsleitung 2 eingefügten Schlauchstückes 6 auf.

Das Schlauchstück 6 ist ein handelsüblicher Zuspritzteil aus einem hochelastischen Elastomer, das üblicherweise in einer PVC-Flüssigkeitsleitung 2 zwischengeschaltet wird, um die Möglichkeit zu bieten, z.B. Medikamente bei einer Infusion oder Transfusion zuzuspritzen. Zum Anschluss an die herkömmliche PVC-Flüssigkeitsleitung 2 überlappt das elastische Schlauchstück 6 mit seinen beiden Enden 63, 64 jeweils die Enden der Flüssigkeitsleitung 2 luftdicht.

Es können auch Verbindungsstutzen eingesetzt werden, wie sie schon an den herkömmlichen Infusionsgeräten verwendet werden. Das Schlauchstück 6 weist in seinem mittleren Teil eine Verdickung 65 auf, die an ihren beiden Enden jeweils in Form einer Ringschulter 61, 62 auf einen zylindrischen Schlauchabschnitt 63, 64 reduziert ist, der etwas grösser ist als der Querschnitt der Flüssigkeitsleitung 2. Diese Verdickung 65 fixiert die Lage der gesamten Dosiervorrichtung 1 an einer bestimmten Stelle in der Flüssigkeitsleitung 2. Sie ermöglicht auch eine Verstärkung der Wandstärke. Dies ist von Bedeutung, wenn das Schlauchstück 6 in der Dosiervorrichtung 1 ebenfalls zum Zuspritzen verwendet werden soll. Andererseits schafft die Verdickung 65 einen möglichst langen freien Weg im Hohlraum 60 des Schlauchstückes 6 für den Einstich einer (nicht dargestellten) Nadelkanüle. Das hochelastische Material des Schlauchstückes 6 gewährleistet, dass sich das Einstichloch nach Durchstechen der Wand des Schlauchstückes 6 mit einer Nadelkanüle und nach Herausziehen dieser Nadelkanüle sofort wieder schliesst.

Das Gehäuse 3 bildet im geschlossenen Zustand eine Kammer 7, in der das Schlauchstück 6 eingefügt ist. Die Kammer 7 ist der Aussenkontur des Schlauchstückes 6 im wesentlichen angepasst und weist im Bereich des dünnen zylindrischen Schlauchabschnittes 63 einen Stutzen 71 und im Bereich des dünnen zylindrischen Schlauchabschnittes 64 einen Kanal 72 auf. Die Ringschultern 61, 62 des Schlauchstückes 6 grenzen jeweils an eine Wand der Kammer 7, so dass die Lage des Schlauchstückes 6 dadurch festgelegt ist. Durch das Gehäuse 3 verläuft in einem spitzen Winkel relativ zur Längsachse des Gehäuses ein Einstichkanal 8 zur Führung einer Nadelkanüle bis zur Kammer 7. Dieser Einstichkanal 8 mündet unmittelbar vor der Ringschulter 61 des Schlauchstückes 6, die durch die stufenförmige Verdickung 65 des Schlauchstückes 6 gebildet wird.

Die Ausführungsbeispiele der Dosiervorrichtung 1 der Fig. 1 bis 8 verwenden als Drückelement einen quaderförmigen Dosierkeil 5, der an seinen breiten Seiten in einer geradlinigen und rechtwinklig zum Schlauchstück verlaufenden Führungsbahn 32 auf den zylindrischen Schlauchabschnitt 64 kurz vor der Ringschulter 61 des Schlauchstückes 6 drückt. Die Stirnfläche 51 des Dosierkeils 5 drückt den Schlauchabschnitt 64 in Richtung auf die ebene Bodenfläche des Kanals 72. Sie verläuft in der Querschnittsebene des Schlauchstückes 6 schräg zu dieser Bodenfläche. Auf diese Weise wird der Schlauchabschnitt 64 bei Zustellen des Dosierkeils 5 von der einen Seite zunehmend geklemmt.

Der Dosierkeil 5 wird von einer Stellvorrichtung 4 betätigt, wobei die Rückstellung aufgrund der elastischen Rückstellkraft des Schlauchstückes 6 erfolgt. Die Stellvorrichtung 5 kann beispielsweise, wie in den Fig. 1 bis 4 dargestellt, eine Gewindespindel 40 sein, die sich in einem Gewinde 33 des Gehäuses 30 befindet und von einem Stellrad 41 betätigt wird. Bei Zustellung der Gewindespindel 40 drückt diese auf den Dosierkeil 5, der seinerseits auf den Schlauchabschnitt 64 drückt. Das Stellrad 41 greift z.B. mit einem Sechskant 42 in eine entsprechende Aussparung 43 im Inneren der Gewindespindel 40. Diese formschlüssige Verbindung ist lösbar und ermöglicht daher ein Abnehmen des Stellrades 41, um z.B. einer unerlaubten Verstellung der Dosiervorrichtung 1 vorzubeugen. Ein mit dem Sechskant 42 koaxial vom Stellrad 41 abstehender Hohlzylinder 44, der sich in eine entsprechende hohlzylindrische Aussparung 34 des Gehäuseteils 3a einfügt, dient zur Führung des Stellrades 41 und verhindert ein Abbrechen des Sechskants 42 durch Verkanten.

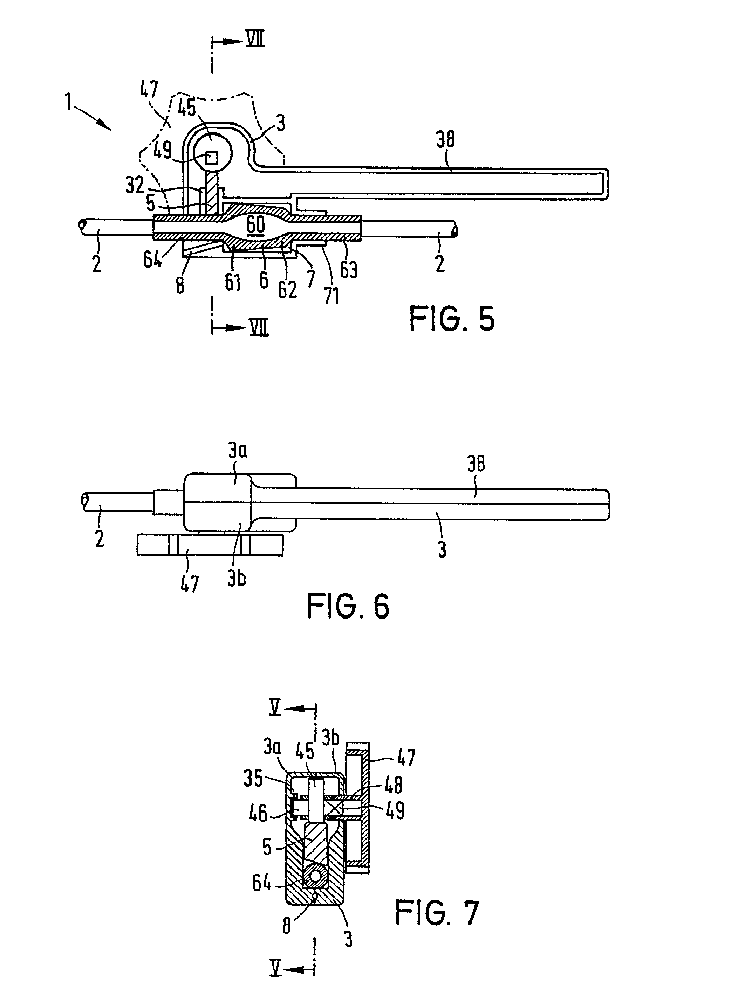

Bei den Ausführungsbeispielen der Fig. 5 bis 8 besteht die Stellvorrichtung 4 aus einem Exzenter 45, der auf einer Exzenterwelle 46 befestigt ist. Die Exzenterwelle 46 wird einerseits von der Lagerung 35 im Gehäuse 3a gehalten, andererseits von einem Schlüsselteil 48 des Stellrades 47 aufgenommen, das seinerseits mit dem Schlüsselteil 48 im Gehäuse 36 gelagert ist. Die Exzenterwelle 46 ist formschlüssig und lösbar mit dem Stellrad 47 verbunden und in ihrem vom Schlüsselteil 48 des Stellrades 47 umfassten Teil 49 z.B. als Vierkant oder Sechskant ausgebildet. Bei Betätigung der Exzenterwelle 46 durch das Stellrad 47 wird der Dosierkeil 5 je nach Winkelstellung des Exzenters 45, der mit seiner äusseren Umfangsfläche auf den Dosierkeil 5 drückt, verstellt. Aufgrund der Elastizität des Schlauchstückes 6 im Schlauchabschnitt 64 wird der Dosierkeil 5 andererseits ständig gegen die äussere Umfangsfläche des Exzenters 45 gedrückt. Diese Stellvorrichtung ist ebenfalls selbsthemmend ausgeführt, so dass das Stellrad 41 sich nicht von selbst verstellen kann. Die übrigen Teile dieser Dosiervorrichtung 1 entsprechen dem vorherigen Ausführungsbeispiel.

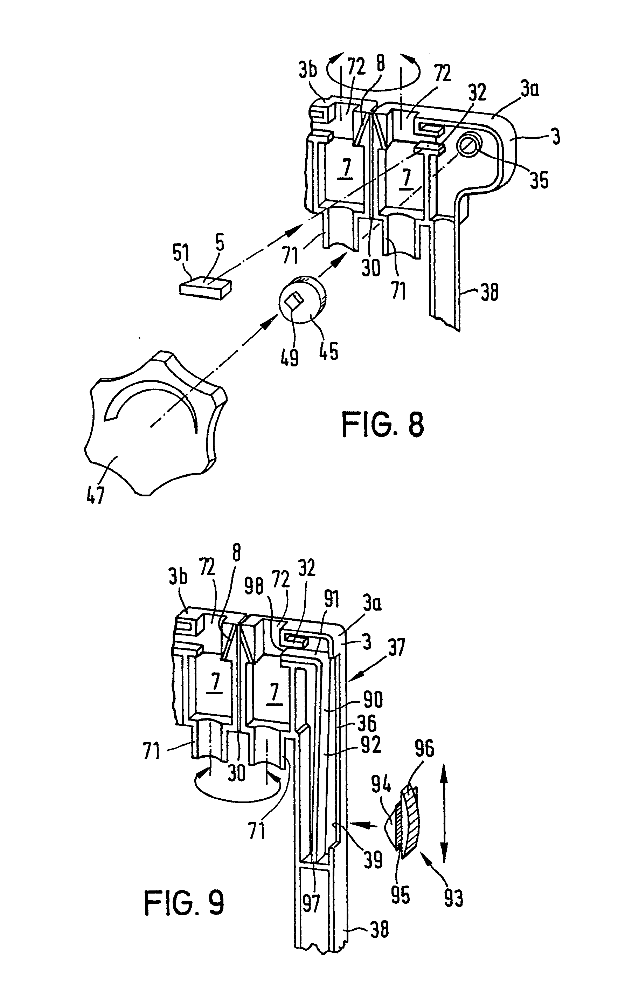

In dem Ausführungsbeispiel gemäss Fig. 9 drückt anstelle des Dosierkeils 5 ein abgewinkelter Hebel 90 mit seinem kurzen Schenkel 91 auf das Schlauchstück 6. Dieser Hebel 90 ist an dem Gehäuse 3a an einem Anlenkpunkt 97 derartig einseitig mit seinem langen Schenkel 92 angelenkt, dass der lange Schenkel 92 im entspannten unverformten Zustand des Schlauchstückes 6 eine zur Gehäusewand 36 geneigte Stellung einnimmt. Zwischen der Gehäusewand 36 und dem langen Schenkel 92 ist der Schieber 93 eingefügt, der sich mit seinem im Gehäuse 3 befindlichen Teil an der Gehäusewand 36 abstützt und dabei auf den langen Schenkel 92 des Hebels 90 drückt. Wird der Schieber 93 vom Anlenkpunkt 97 des Hebels 90 wegbewegt, drückt er aufgrund der Neigung des Hebels 90 wie ein Keil zunehmend auf den langen Schenkel 92, so dass der nahezu im rechten Winkel abgewinkelte kurze Schenkel 91 des Hebels 90 zunehmend auf das Schlauchstück 6 drückt. Die auf das Schlauchstück 6 wirkende Stirnseite 98 des kurzen Schenkels 91 des Hebels 90 ist entsprechend dem Dosierkeil 5 quer zur Schlauchrichtung abgeschrägt, so dass das Schlauchstück 6 zunehmend von einer Seite geklemmt wird, wenn der Hebel 90 in die Schliessposition geschwenkt wird. Der Schieber 93 wird in einem Schlitz 37 in der oberen Gehäusewand 36 geführt. Die Innenseite 39 der Gehäusewand 36 ist gerippt, damit in Verbindung mit der verrippten Oberfläche 95 des im Gehäuse 3 befindlichen Teils 94 des Schiebers 93 eine Selbsthemmung der Stellvorrichtung erzielt wird.

Der obere Teil 96 des Schiebers 93 kann entsprechend den Stellrädern 41, 47 der vorherigen Ausführungsbeispiele ebenfalls abnehmbar sein, um eine unerlaubte Betätigung der Dosiervorrichtung 1 durch Unbefugte zu verhindern.

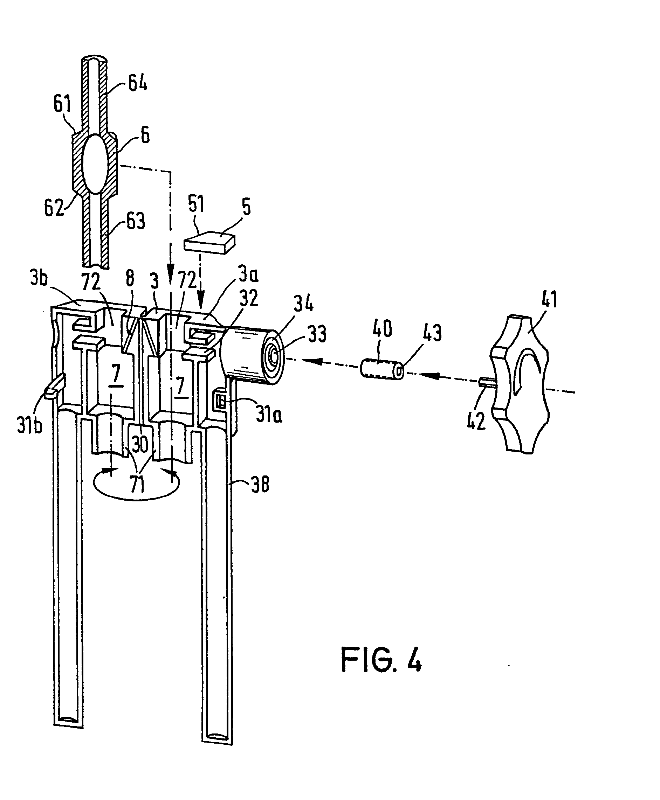

Das Gehäuse 3 wird als Spritzgussteil in einem Stück, z.B. aus Polypropylen, hergestellt. Alle dargestellten Gehäuse sind so gestaltet, dass sie in Längsrichtung zwei z.B. um ein Filmscharnier 39 schwenkbare Gehäuseteile 3a, 3b aufweisen (Fig. 4, 8, 9). Die beiden Gehäuseteile 3a, 3b lassen sich durch Zusammendrücken mit Hilfe von Schnappverbindungen 31a, 31b miteinander arretieren. Nach dem Zusammendrücken der beiden Gehäuseteile 3a, 3b ist die Verbindung zerstörungsfrei nicht mehr lösbar. Die beiden Gehäuseteile weisen ein parallel zur Flüssigkeitsleitung 2 verlaufendes Griffstück 38 auf, das eine Einhandbedienung der Stellvorrichtung 4 in Kombination mit einem Stellrad 41, 47 bzw. einem Schieber 93 ermöglicht. Die Stellräder 41, 47 bzw. der Schieber 93 sind dabei in ihrer Aussenkontur so geformt, dass sie leicht mit dem Daumen betätigt werden können, während die anderen Finger der Hand das Griffstück 38 am Gehäuse 3 umfassen.

### Patentansprüche

1. Dosiervorrichtung für Flüssigkeiten für Infusions- oder Transfusionsgeräte mit einer durch ein Gehäuse (3) hindurchgehenden flexiblen Flüssigkeitsleitung (2), die im Inneren des Gehäuses (3) ein Schlauchstück (6) aus hochelastischem elastomerem Material enthält, und mit einem am Gehäuse (3) abgestützten Drückelement (5) zum Zusammendrücken des Schlauchstückes (6), dadurch gekennzeichnet, dass das Schlauchstück (6) auf einem Teil seiner Länge eine Verdickung aufweist, dass das Gehäuse (3) an der Verdickung (65) des Schlauchstückes (6) gegen Längsverschiebung gesichert ist und dass das Drückelement (5) ausserhalb des Bereiches der Verdickung (65) an dem Schlauchstück (6) angreift.

2. Dosiervorrichtung nach Anspruch 1, dadurch gekennzeichnet, dass das Gehäuse (3) einen zum Schlauchstück (6) führenden Einstichkanal (8) für eine Nadelkanüle aufweist.

3. Dosiervorrichtung nach Anspruch 2, dadurch gekennzeichnet, dass der Einstichkanal (8) unter einem spitzen Winkel zur Längsachse des Schlauchstückes (6) verläuft.

4. Dosiervorrichtung nach Anspruch 3, dadurch gekennzeichnet, dass der Einstichkanal (8) an einer Ringschulter (61) an einem Ende der Verdickung (65) mündet.

5. Dosiervorrichtung nach einem der Ansprüche 1 bis 4, dadurch gekennzeichnet, dass das Drückelement (5) senkrecht zur Längsrichtung des Schlauchstückes (6) geführt ist und dass die gegen das Schlauchstück (6) drückende Stirnfläche (51) des Drückelementes (5) zur Bewegungsrichtung des Drückelementes (5) schräggestellt ist.

6. Dosiervorrichtung nach einem der vorhergehenden Ansprüche, dadurch gekennzeichnet, dass das Drückelement (5) über eine manauell verstellbare selbsthemmende Stellvorrichtung (4) bewegbar ist.

7. Dosiervorrichtung nach Anspruch 6, dadurch gekennzeichnet, dass die Stellvorrichtung (4) aus einer in dem Gehäuse (3) gelagerten Gewindespindel (40) besteht.

8. Dosiervorrichtung nach Anspruch 6, dadurch gekennzeichnet, dass die Stellvorrichtung (4) aus einem an dem Gehäuse (3) gelagerten Exzenter (45) besteht.

9. Dosiervorrichtung nach Anspruch 6, dadurch gekennzeichnet, dass die Stellvorrichtung (4) aus einem Hebel (90) besteht, der um einen Anlenkpunkt (97) im Gehäuseinneren schwenkbar ist und mit einem freien Hebelende (91) als Drückelement (5) auf das Schlauchstück drückt, dass in einem Schlitz (37) einer Wand (36) des Gehäuses (3) ein Schieber (93) geführt ist und dass der Hebel (90) in bezug auf die den Schlitz (37) enthaltende Wand (36) derart schräggestellt ist, dass er bei einer Längsbewegung des Schiebers (93) in dem Schlitz (37) zunehmend verschwenkt wird.

10. Dosiervorrichtung nach einem der Ansprüche 6 bis 9, dadurch gekennzeichnet, dass die Stellvorrichtung ein abnehmbares Stellteil (41, 47, 93) aufweist.

### Claims

1. Liquid metering device for infusion or transfusion apparatuses comprising a flexible fluid line (2) extending through a housing (3) and containing inside the housing (3) a hose member (6) of highly flexible elastomeric material, and a pressing element (5) supported at housing (3) for compressing the hose member (6), characterized in that the hose member (6) contains on part of its length a thicker portion, that the housing (3) is protected against longitudinal displacement by the thicker portion (65) of the hose member (6) and that the pressing element (5) engages the hose member (6) outside the region of the thicker portion (65).

2. Metering device according to claim 1, characterized in that the housing (3) comprises a puncture channel leading to the hose member (6) for introducing a needle canula.

3. Metering device according to claim 2, characterized in that the puncture channel (8) extends at an acute angle relative to the longitudinal axis of the hose member (6).

4. Metering device according to claim 3, characterized in that the puncture channel (8) ends at an annular shoulder (61) formed on one end of the thicker portion (65).

5. Metering device according to one of claims 1 to 4, characterized in that the pressing element (5) is directed perpendicular relative to the longitudinal direction of the hose member (6) and that the end face (51) of the pressing element (5) urging against the hose member (6) is inclined relative to the moving direction of the pressing element (5).

6. Metering device according to one of the preceding claims, characterized in that the pressing element (5) is movable via a manually adjustable self-locking setting means (47).

7. Metering device according to claim 6, characterized in that the setting means (4) consists of a threaded spindle (40) supported in housing (3).

8. Metering device according to claim 6, characterized in that the setting means (4) consists of an eccentric (45) supported at housing (3).

9. Metering device according to claim 6, characterized in that the setting means (4) is a lever (90) adapted to swivel about a pivot point (97) inside the housing and pressing with a free end (91) as a pressing element (5) on the hose member, that in a slot (37) of a wall (36) of housing (3), a slide (93) is guided and that the lever (90) is so inclined relative to the wall (36) containing the slot (37) that, in case of a longitudinal movement of the slide (93), it is gradually swivelled in the slot (37).

10. Metering device according to one of claims 6 to 9, characterized in that the setting means contains a detachable setting element (41, 47, 93).

**Revendications**

1. Dispositif de dosage pour liquides destinés à des appareils d'infusion ou de transfusion, comportant une tubulure de liquide (2) flexible et traversant de part en part un boîtier (3) et qui comporte, à l'intérieur du boîtier (3), un tronçon de tuyau (6) en une matière élastomère à haute élasticité, et comportant un élément de pression (5) s'appuyant sur le carter (3) et servant à comprimer le tronçon de tuyau (6), caractérisé en ce que le tronçon de tuyau (6) présente sur une partie de sa longueur un épaississement, en ce que le boîtier (3) est immobilisé à l'encontre d'un déplacement longitudinal, sur l'épaississement (65) du tronçon de tuyau (6), et en ce que l'élément de pression (5) est en prise sur le tronçon de tuyau (6) à l'extérieur de la zone de l'épaississement (65).

2. Dispositif de dosage selon la revendication 1, caractérisé en ce que le boîtier (3) comporte un canal de piquage (8) conduisant au tronçon de tuyau (6) et destiné à une canule à aiguille.

3. Dispositif de dosage selon la revendication 2, caractérisé en ce que le canal de piquage (8) s'étend sous un angle aigu par rapport à l'axe longitudinal du tronçon de tuyau (6).

4. Dispositif de dosage selon la revendication 3, caractérisé en ce que le canal de piquage (8) débouche sur un épaulement annulaire (61) situé à une extrémité de l'épaississement (65).

5. Dispositif de dosage selon l'une des revendications 1 à 4, caractérisé en ce que l'élément de pression (5) est guidé perpendiculairement à la direction longitudinale du tronçon de tuyau (6) et en ce que la face frontale (51) de l'élément de pression (5) qui applique une pression contre le tronçon de tuyau (6) est disposée inclinée par rapport à la direction de déplacement de l'élément de pression (5).

6. Dispositif de dosage selon l'une des revendications précédentes, caractérisé en ce que l'élément de pression (5) est déplaçable à l'aide d'un dispositif de réglage (4) à autoblocage et réglable manuellement.

7. Dispositif de dosage selon la revendication 6, caractérisé en ce que le dispositif de réglage (4) est constitué d'une tige filetée (40) montée à rotation dans le boîtier (3).

8. Dispositif de dosage selon la revendication 6, caractérisé en ce que le dispositif de réglage (4) est constitué d'un excentrique (45) monté à rotation sur le boîtier (3).

9. Dispositif de dosage selon la revendication 6, caractérisé en ce que le dispositif de réglage (4) est constitué d'un levier (90) qui peut basculer autour d'un point d'articulation (97) se trouvant à l'intérieur du boîtier et qui applique une pression sur le tronçon de tuyau à l'aide d'une extrémité libre de levier (91) d'élément de pression (5), en ce qu'un poussoir (93) est guidé dans une fente (37) d'une paroi (36) du boîtier (3) et en ce que le levier (90) est disposé incliné par rapport à la paroi (36) contenant la fente (37) de manière qu'il bascule progressivement lors d'un déplacement longitudinal du poussoir (93) dans la fente (37).

10. Dispositif de dosage selon l'une des revendications 6 à 9, caractérisé en ce que le dispositif de réglage comporte une partie amovible de réglage (41, 47, 93).

FIG. 1

FIG. 2

FIG. 3

FIG. 4

FIG. 5

FIG. 6

FIG. 7

**FIG. 8**

**FIG. 9**